# EUROPEAN PATENT APPLICATION

(11) **EP 4 527 332 A1**
(43) Date of publication of application: **26.03.2025**
(21) Application number: 24201695.4
(22) Date of filing: 20.09.2024
(51) Int. Cl.: A61B 18/14, A61B 5/00, A61B 18/00

(54) **MEDICAL DEVICE WITH A MONOLITHIC SPINE FRAMEWORK**

(30) Priority: 22.09.2023 US 202318473169
(71) Applicant: Biosense Webster (Israel) Ltd., 2066717 Yokneam (IL)
(72) Inventor: PHAM, Cuong, Irvine, 92618 (US); SOLTE, Isabel Barbero, Irvine, 92618 (US); JOSHI, Helee Mukul, Irvine, 92618 (US); NGUYEN, Thanh, Irvine, 92618 (US); DATTA, Keshava, Irvine, 92618 (US)
(74) Representative: Carpmaels & Ransford LLP

(57) **Abstract**

The disclosed technology includes monolithic framework for an end effector of a medical device. The monolithic framework includes cylindrical stock. The cylindrical stock includes a crown and multiple spines. The crown extends along a longitudinal axis, defines a crown lumen, and defines a plurality of crown recesses that, in use, connect to an annulus. The plurality of spines also extend along the longitudinal axis and bow outwardly relative to the longitudinal axis. Each spine extends from the crown to a spine terminal end and, in use, connects to a connecting hub.

## Description

### FIELD

The present technology relates generally to medical devices, and in particular medical probes with electrodes, and further relates to, but not exclusively, medical probes suitable for use to map and/or ablate tissue.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

Basket-style catheters are typically formed from multiple pieces of metal cut from a sheet, which requires assembly to form the basket. Moreover, typical basket catheters provide a spheroidal configuration only when expanded. Accordingly, there is a need for improved techniques to form an end effector of a basket catheter with fewer components as well as different configurations in the expanded and collapsed configurations.

### SUMMARY

There is provided, in accordance with the disclosed technology, a monolithic framework for an end effector of a medical device. The monolithic framework can include cylindrical stock. The cylindrical stock can include a crown and a plurality of spines. The crown extends along a longitudinal axis, defines a crown lumen, and defines a plurality of crown recesses that are configured to connect to an annulus. The spines extend along the longitudinal axis and are configured to bow outwardly relative to the longitudinal axis. Each spine extends from the crown to a spine terminal end and is configured to connect to a connecting hub.

There is further provided, in accordance with the disclosed technology, an end effector for a medical device. The end effector can include a monolithic framework, an annulus, a connecting hub, and one or more electrodes. The monolithic framework can include cylindrical stock that includes a crown and a plurality of spines. The crown extends along a longitudinal axis and defines a plurality of crown recesses. The spines extend along the longitudinal axis and are configured to bow outwardly relative to the longitudinal axis. The spines are movable between a collapsed configuration and an expanded configuration. The annulus is connected to the crown recesses. The connecting hub is connected to the spines. The one or more electrodes is connected to each spine.

There is further provided, in accordance with the disclosed technology, a method of manufacturing a monolithic framework for a medical device. The method can include the following steps. A generally cylindrical stock is formed to include a crown and a plurality of spines. The crown extends along a longitudinal axis, defines a crown lumen that is aligned with a stock lumen defined by the generally cylindrical stock, and defines a plurality of crown recesses configured to connect to an annulus. The spines extend along the longitudinal axis. Each spine extends from the crown to a spine terminal end and is configured to connect to a connecting hub. The spines are heat set such that they bow outwardly relative to the longitudinal axis.

### BRIEF DESCRIPTION OF THE DRAWINGS

FIG. 1 is a schematic pictorial illustration of a medical system including a medical device with a distal end that includes a medical probe with electrodes, in accordance with the disclosed technology;
FIG. 2 is a schematic pictorial illustration showing a perspective view of a distal tip of the medical device, such as a medical probe, in accordance with the disclosed technology;
FIG. 3 is a schematic pictorial illustration showing a perspective view of an end effector, with a sleeve and coil removed for clarity, in accordance with the disclosed technology;
FIG. 4A is a schematic pictorial illustration showing a detail perspective view of a framework distal end of a monolithic framework, with an annulus detached therefrom, in accordance with the disclosed technology;
FIG. 4B is a schematic pictorial illustration showing a detail perspective view of the framework distal end with the annulus partially assembled thereto, in accordance with the disclosed technology;
FIG. 4C is a schematic pictorial illustration showing a detail perspective view of the framework distal end with the annulus partially assembled thereto, in accordance with the disclosed technology;
FIG. 5 is a schematic pictorial illustration showing a cross-sectional view of the framework distal end and the annulus, cut relative to line 5-5 in FIG. 4C, in accordance with the disclosed technology;
FIG. 6A is a schematic pictorial illustration showing a detail perspective view of a proximal end of the end effector, with a connecting hub detached from the proximal end, in accordance with the disclosed technology;
FIG. 6B is a schematic pictorial illustration showing a detail perspective view of the proximal end of the end effector, with the connecting hub connected to the framework proximal end 102B, in accordance with the disclosed technology;
FIG. 7 is a schematic pictorial illustration showing a cross-sectional view of the framework proximal end connected to the connecting hub, cut relative to line 7-7 in FIG. 6B, in accordance with the disclosed technology;
FIG. 8 is a schematic pictorial illustration showing a generally cylindrical stock formed into a monolithic framework, with a plurality of jackets formed and/or positioned over the spines of the framework, in accordance with the disclosed technology;
FIG. 9 is a flow chart showing a method of manufacturing the monolithic framework in accordance with the generalized procedure shown in FIG. 8, in accordance with the disclosed technology;
FIG. 10 is a schematic pictorial illustration showing a perspective view of another configuration of end effector of a medical device, in accordance with the disclosed technology;
FIG. 11 is a schematic pictorial illustration showing a perspective view of the end effector of FIG. 10, with a proximal sleeve and coil removed for clarity, in accordance with the disclosed technology; and
FIG. 12 is a schematic pictorial illustration showing a cross-sectional view of the end effector of FIG. 10, cut relative to line 12-12 in FIG. 10.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for irreversible electroporation (IRE), RF ablation, and/or cryoablation. IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The technology of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

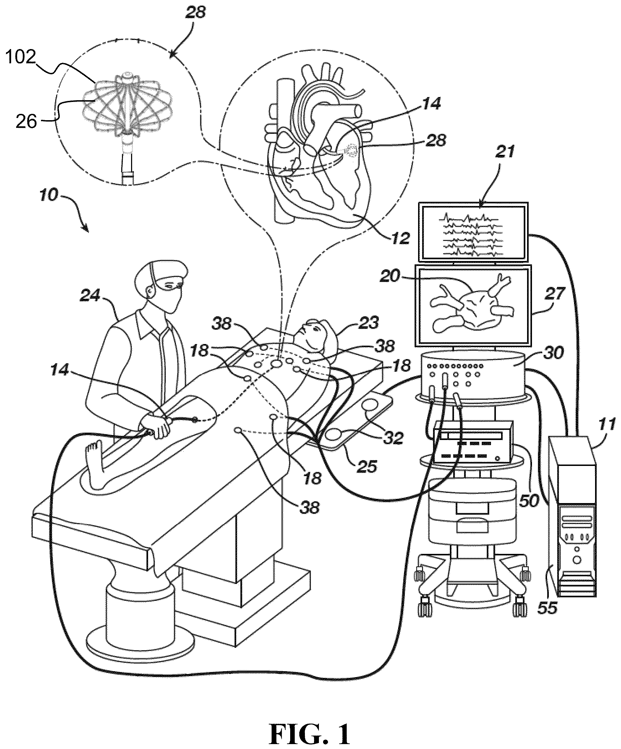

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example medical device/probe, e.g., a catheter 14, that is configured for sensing IEGM is illustrated herein. Physician 24 brings a distal tip of catheter 14 (i.e., a basket assembly 28 in this case) into contact with the heart wall for sensing a target site in heart 12. A catheter 14 with a distal basket assembly can be referred to as a basket catheter. For ablation, physician 24 would similarly bring a distal end of an ablation catheter to a target site for ablating.

Catheter 14 is an exemplary catheter that includes one and preferably multiple electrodes 26 optionally distributed over a plurality of spines 104 at basket assembly 28 and configured to sense the IEGM signals. In examples described herein, electrodes 26 can be configured to deliver ablation energy (IRE and/or RF) to tissue in heart 12. In addition to using electrodes 26 to deliver ablation energy, the electrodes 26 can also be used to determine the location of the end effector 100 and/or to measure a physiological property such as local surface electrical potentials at respective locations on tissue in heart 12. The electrodes 26 can be biased such that a greater portion of the electrode 26 faces outwardly from the end effector 100 such that the electrodes 26 deliver a greater amount of electrical energy outwardly away from the end effector 100 (i.e., toward the heart 12 tissue) than inwardly toward the end effector 100.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

Catheter 14 may additionally include a position sensor embedded in or near basket assembly 28 for tracking position and orientation of basket assembly 28. Optionally and preferably, position sensor is a magnetic based position sensor including three magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of basket assembly 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

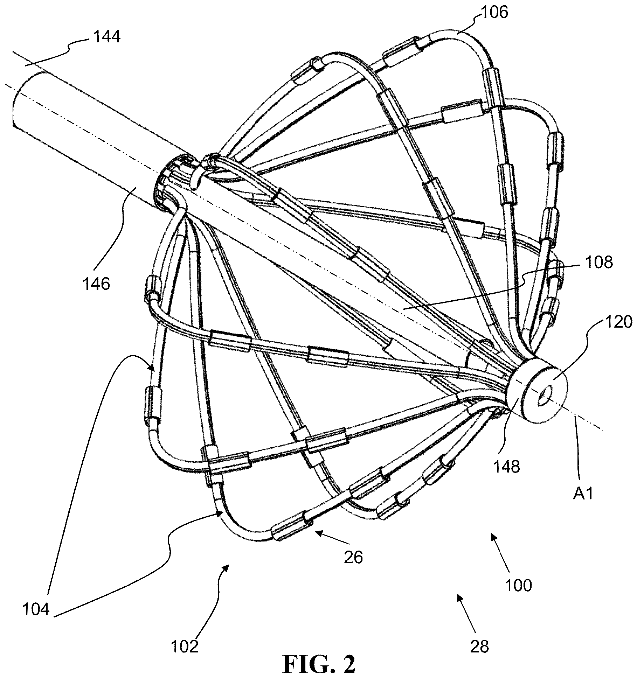

FIG. 2 is a schematic pictorial illustration showing a perspective view of a distal tip 28 of a medical device 14, such as a medical probe. The medical device 14 includes, at its distal tip 28, an end effector 100. The end effector 100 in the presently described example takes the form of a basket assembly that includes at least one spine 104. Spines 104 may have elliptical (e.g., circular) or rectangular (that may appear to be flat) cross-sections, and include a flexible, resilient material (e.g., a shape-memory alloy such as nickel-titanium, also known as Nitinol) forming a strut.

The end effector 100 is connected to the rest of the medical probe 14 via an elongated shaft or tube 144. The elongated shaft 144 can be tubular in form and flexible, with certain portions being more flexible than others. For example, a tip portion can be made more flexible than the rest to allow the end effector 100 to be easily deflected. The elongated shaft 144 can be formed from a flexible, biocompatible electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3 -hydroxyvalerate) (PHBV), poly-L-lactide, polydioxanone, polycarbonates, and polyanhydrides with the ratio of certain polymers being selected to control the degree of inflammatory response.

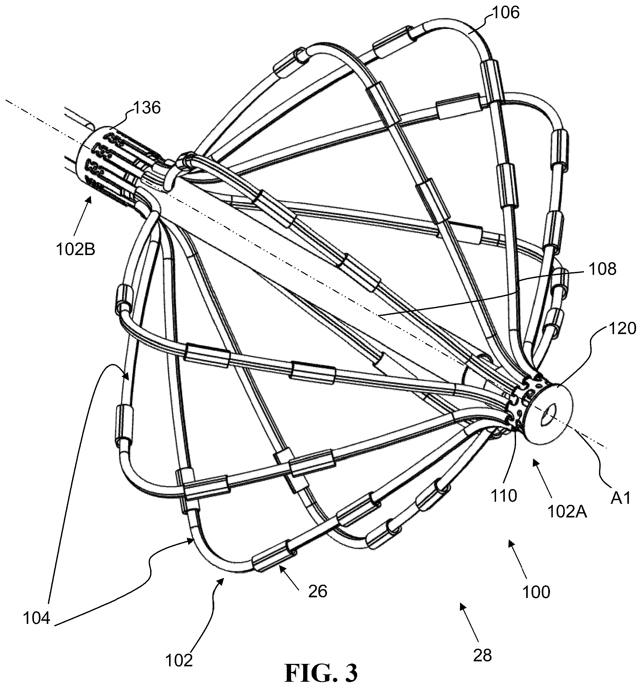

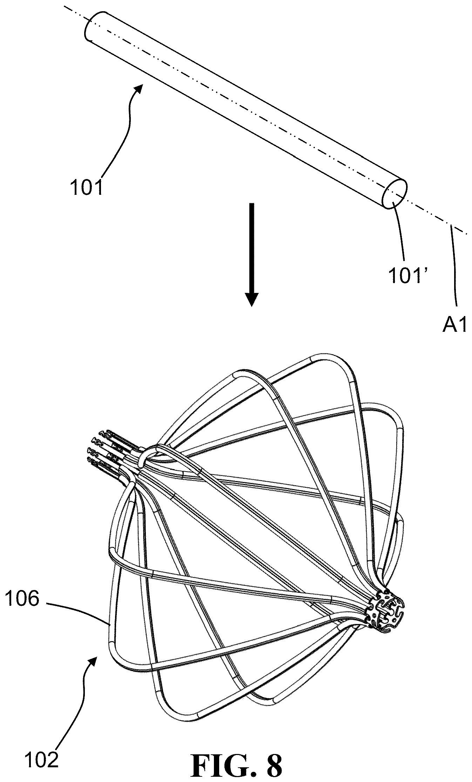

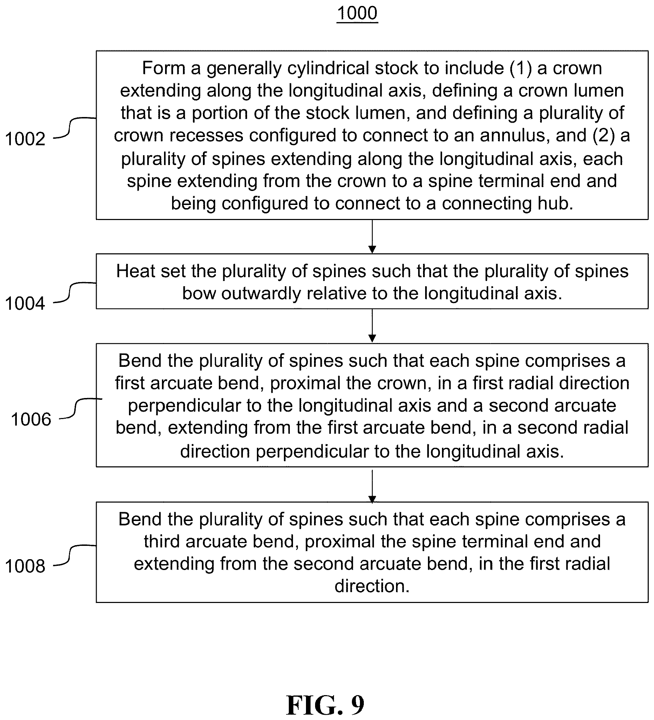

The spines 104 may be formed integrally with a distal hub (referred to herein as crown 110), as seen in FIG. 3, so as to form a unitary/monolithic spine framework 102. The crown 110 extends along a longitudinal axis A1. The framework 104 can be formed from a planar or cylindrical tube stock of material using any suitable method. For example, the framework 102 can be formed by cutting, laser cutting, stamping, combinations thereof, etc. Further discussion on a method of manufacturing the framework is provided below with respect to FIGs. 8 and 9.

An atraumatic annulus 120 and coil 148 are connected to the crown 110. In some examples, the coil 116 can comprise a single axis sensor (SAS). In other examples, the coil 116 can comprise a dual axis sensor (DAS) or a triple axis sensor (TAS). The coil 116 can comprise a conductive material wound in a coil or a coil formed into a flexible circuit. The coil 116 can comprise electrical leads for conduction of current induced on the coil to the patient interface unit 30. As will be appreciated, by attaching a coil 116 to the distal end of the end effector 100, it is possible to detect a position thereof. In this way, a physician 24 can more accurately determine the position of the distal end of the end effector 100 before applying ablative energy to tissue.

An insulative material, referred to herein as a jacket 106, is provided over each spine. Each jacket 106 serves to enhance the atraumaticity of the end effector 100 to protect the subject from sharp edges and for irrigation purposes described in greater detail below. The jacket 106 can include a polymer. For example, the jacket 106 can be formed from a flexible, biocompatible electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Furthermore, while the jacket 106 is shown to be tubular in these figures, the jacket 106 can be shaped, scalloped, ribbed, ridged, concaved, convexed, or otherwise configured such that the overall profile of jacket 106 yields physical and/or mechanical properties, such as rigidity and flexion along multiple axes, required by the end effector 100, mentioned above. Moreover, a flexible sleeve 146 is provided over proximal end of the end effector 100 and an irrigation manifold 120 (FIG. 3), which is discussed in greater detail below.

The spine(s) 104 are movable between an expanded configuration and a collapsed configuration. The elongated shaft 144 connects the end effector 100 to a handle that, in use, the operator 24 can manipulate. A connector tube 108 is connected to the crown 110. In some examples, the connector tube 108 is used to facilitate the expanding and collapsing of the spines 104. For example, the connector tube 108 can be translated along the longitudinal axis A1 to expand/collapse the spines 104. In the expanded configuration (FIG. 2) as well as the collapsed configuration, one or more spines 104 can bow radially outwardly from the longitudinal axis A1. The connector tube 108 can additionally or alternatively be used to route irrigation fluid therethrough. The spines 104, elongated shaft 144, crown 110, and connector tube 108 are arranged generally along the longitudinal axis A1 when the elongated shaft 144 is unbent. Moreover, a sleeve 146 is provided over a proximal end of the end effector 100 increase the atraumaticity thereof.

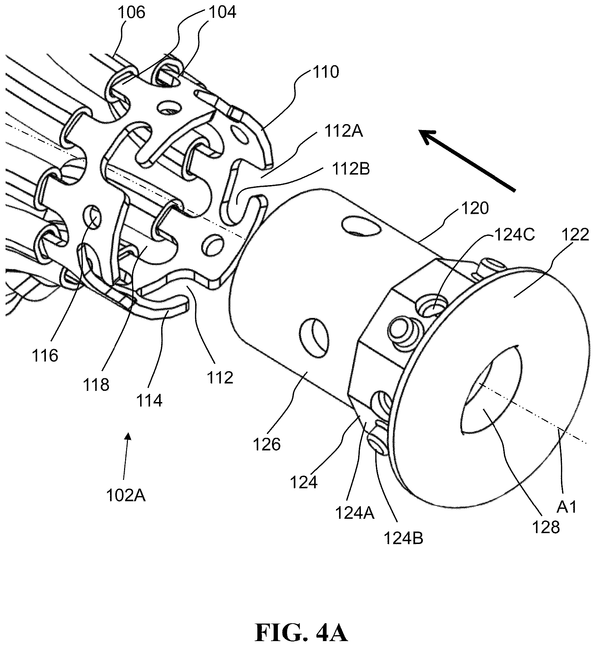

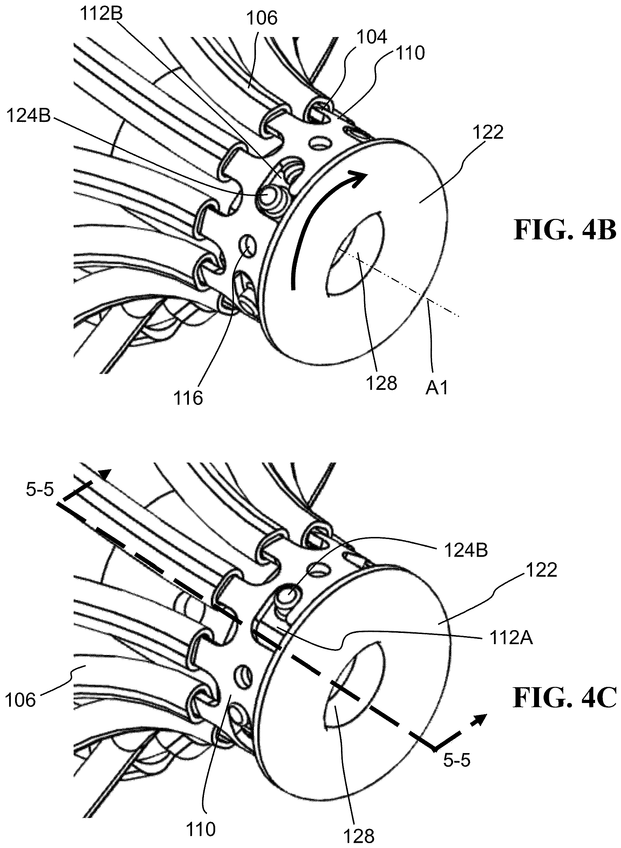

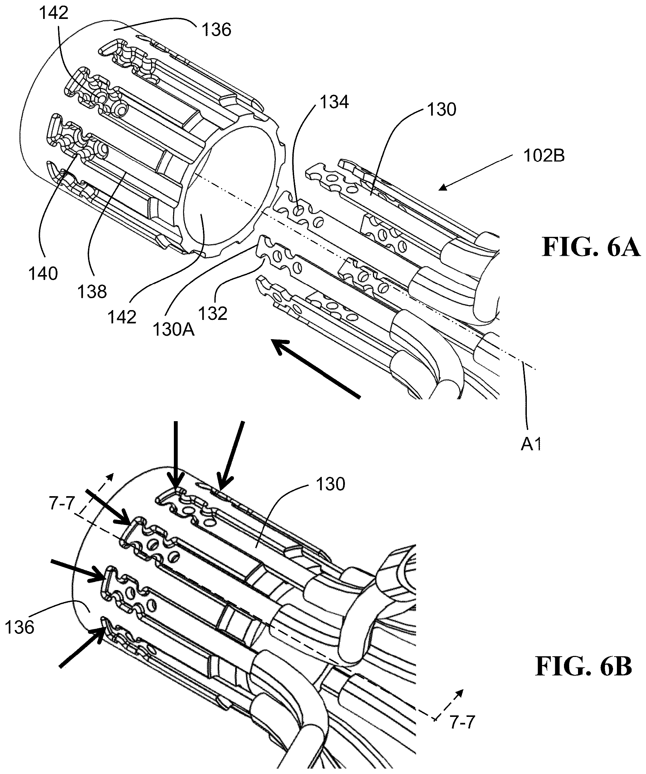

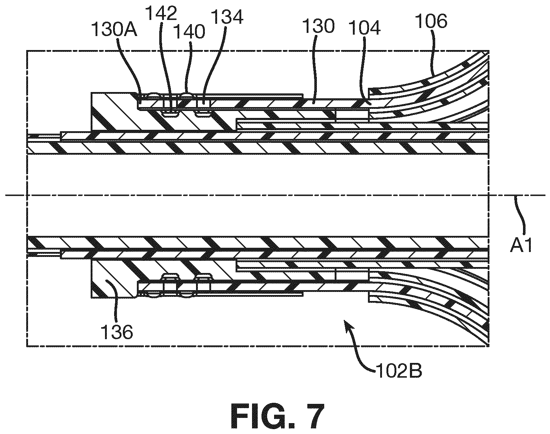

FIG. 3 is a schematic pictorial illustration showing a perspective view of the end effector 100, with the sleeve 146 and coil 116 removed for clarity. As shown, the framework 102 has a distal end 102A (that includes the crown 110) and a proximal end 102B. The crown 110 connects with the annulus 120 in a manner discussed in greater detail below with respect to FIGs. 4A, 4B, and 4C. The proximal end 102B is connected with a connecting hub 136 and covered with the sleeve 146 to, as mentioned above, increase the atraumaticity of the end effector 100. The connecting hub 136 can be substantially cylindrical in form and maintains the positions of the ends of the spines 104 with respect to one another, and is discussed in greater detail with respect to FIGs. 6A, 6B, and 7.

FIG. 4A is a schematic pictorial illustration showing a detail perspective view of the framework distal end 102A with the annulus 120 detached therefrom. FIG. 4B is a schematic pictorial illustration showing a detail perspective view of the framework distal end 102A with the annulus 120 partially assembled thereto. FIG. 4C is a schematic pictorial illustration showing a detail perspective view of the framework distal end 102A with the annulus 120 partially assembled thereto.

As shown in FIG. 4A, the crown 110 defines a crown lumen 118 coaxial and/or parallel with the longitudinal axis A1. Additionally, defined in a terminal end 114 of the crown 110 are a plurality of crown recesses 112 disposed circumferentially about the longitudinal axis A1. In some examples, the recesses 112 have an entry section 112A and a receiving section 112B, extending generally perpendicular to the entry section, that form an L-shape to lock the annulus 120, which is discussed in greater detail below. Further, between adjacent L-shaped recesses 112 are a plurality of cylindrical crown holes 116.

With continued reference to FIG. 4A, the annulus 120 is generally tubular in form and includes an atraumatic tip 122, a connecting portion 124 connected to the tip 122, a tube portion 126 connected to the connecting portion 124, and a lumen 128 therethrough. The lumen 128 permits various medical devices, such as a guidewire and/or mapping catheter, to pass through the annulus 120. The connecting portion 124 includes adjacent/proximal sets of locking protrusions 124B and annulus holes 124C. Further, the connecting portion 124 includes a polygonal cross-sectional shape.

As depicted in the sequence from FIG. 4A to FIG. 4B to FIG. 4C, the crown 110 and annulus 120 have complementary designs to allow easy fixation of the annulus 120 to the crown 110. As denotated by the directional arrow in FIG. 4A, the tube portion 126 and the connecting portion 124 are inserted into the crown lumen 118 when being assembled. As this occurs, the locking protrusions 124B enter the recesses 112 through the entry section 112A (FIG. 4B). To lock the annulus 120 to the crown 110, the annulus 120 is rotated about the longitudinal axis A1 (e.g., clockwise), as denoted by the directional arrow in FIG. 4B. Finally, FIG. 4C depicts the annulus 120 following rotation thereof to a locking position. The polygonal connecting portion 124 frictionally engages an inner surface of the crown 110 to aid in retention. To disassemble, the reverse steps can be employed.

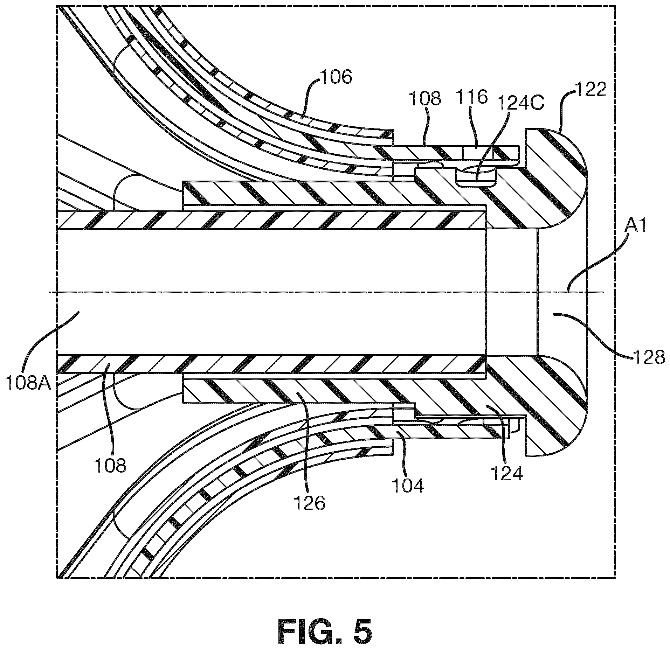

FIG. 5 is a schematic pictorial illustration showing a cross-sectional view of the framework distal end 102A and the annulus 120 cut relative to line 5-5 in FIG. 4C. When the annulus 120 is rotated to the locking position, the crown holes 116 align with the annulus holes 124C. This alignment enables a fastener, e.g., a screw, a rivet, a solder, or the like, to pass through the crown hole 116 to connect with the annulus hole 124C. As also seen in this view, the connector tube 108 (which defines a connector tube lumen 108A) slides within the tube portion 126 to axially align with the annulus 120.

FIG. 6A is a schematic pictorial illustration showing a detail perspective view of a proximal end of the end effector 100, with the connecting hub 136 detached from the framework proximal end 102B. FIG. 6B is a schematic pictorial illustration showing a detail perspective view of a proximal end of the end effector 100, with the connecting hub 136 connected to the framework proximal end 102B. Further to the above, and making reference to FIGs. 6A and 6B, the spines 104 have proximal ends 130 that are shaped to connect to the connecting hub 136. Specifically, each spine 104 defines a plurality of spine recesses 132 in at least one lateral side of the spine 104 (e.g., the recesses 132 can be defined in both lateral sides of the spine 104, as shown). Further, at least one spine hole 134 is defined through each spine end 130 that is directed perpendicularly to the longitudinal axis A1. As seen particularly in FIG. 6A, the spine holes 134 are disposed between pairs/sets of the spine recesses 132 defined on the opposing lateral sides of the spine proximal ends 130. The connecting hub 136 includes complementarily shaped hub reliefs 138 that are shaped to receive a respective shaped proximal end 130 of the spines 130. Each hub relief includes one or more relief protrusions 140 and one or more relief holes 142. In particular, the spine recesses 132 interface with the relief protrusions 140 when the proximal ends 130 are set into place. FIG. 6B illustrates, as denoted by the directional arrows, the proximal ends 130 being aligned and moved radially inwardly towards the longitudinal axis A1 (compare with FIG. 6A which depicts A1) to connect with the connecting hub reliefs 138.

FIG. 7 is a schematic pictorial illustration showing a cross-sectional view of the framework proximal end 102B connected to the connecting hub 136, cut relative to line 7-7 in FIG. 6B. As shown, when the spine distal proximal ends 130 are inserted into the hub reliefs 138, the spine holes 134 axially align with the relief holes 142. This alignment enables a fastener, e.g., a screw, a rivet, a solder, or the like, to pass through the crown hole spines hole 134 to connect with the respective relief hole 142.

FIG. 8 is a schematic pictorial illustration showing a generally cylindrical stock 101 formed into a monolithic framework 102, with a plurality of jackets 104 formed and/or positioned over the spines 104 of the framework 102. FIG. 9 is a flow chart showing a method 1000 of manufacturing the monolithic framework 102 in accordance with the generalized procedure shown in FIG. 8.

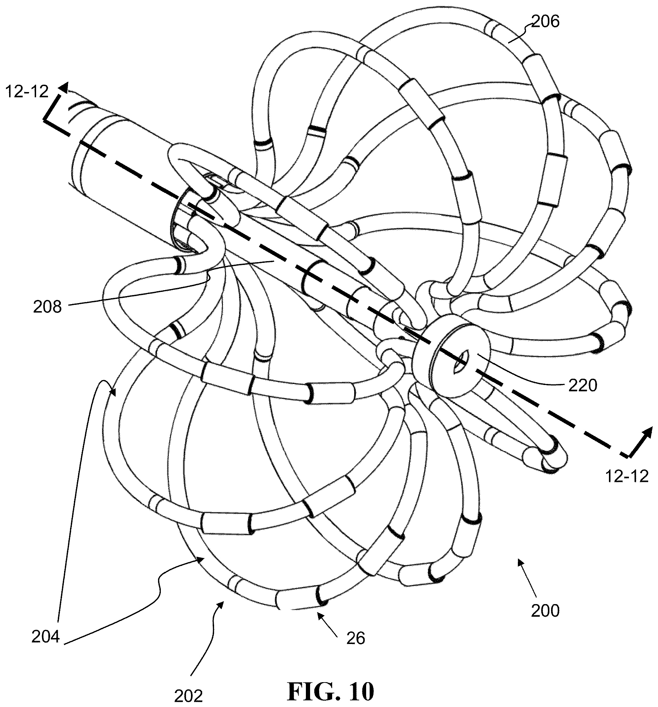

Further to the above, and making reference to FIGs. 8 and 9, a method of manufacturing the monolithic framework 102 may include the following steps. A generally cylindrical stock 101 (see the upper portion of FIG. 8) is formed to include a crown 110 extending along the longitudinal axis A1, defining a crown lumen 118 that is contiguous with a portion of the stock lumen 101', and defining a plurality of crown recesses 112 configured to connect to an annulus 120 (Step 1002). The generally cylindrical stock is also formed to include a plurality of spines 104 extending along the longitudinal axis A1, each spine 104 extending from the crown 110 to a spine terminal end 130 and being configured to connect to a connecting hub 136 (Step 1002). The spines 104 are heat set such that they spine 104 bow outwardly relative to the longitudinal axis A1 (see the lower portion of FIG. 8) (Step 1004). The forming step to form the crown 110 and/or the spines 104 includes at least one of mechanical cutting, laser cutting, stamping, or combinations thereof. For example, the generally cylindrical stock 101 can be cut along the longitudinal axis A1 to form the spines 104, and the distal end of the stock 101 (which is not cut as how the spines 104 are formed) can be cut to define the previously discussed crown recesses 112 as well as crown holes 116. Steps 1006 and 1008 are discussed in greater detail with respect to FIGs. 10-12 below.

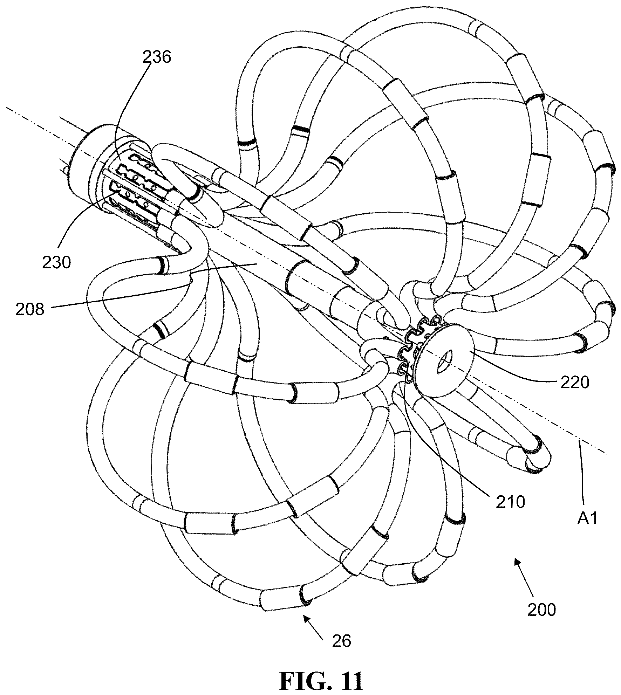

FIG. 10 is a schematic pictorial illustration showing a perspective view of another configuration of end effector 200 of a medical device 14. FIG. 11 is a schematic pictorial illustration showing a perspective view of the end effector 200, with a proximal sleeve and coil removed for clarity (similar to FIG. 3 in relation to FIG. 2 for the first configuration of end effector 100). Features not shared with the previously described end effector 100 are emphasized in the following description.

The end effector 200 in the presently described example, like the example of FIGs. 2-7, takes the form of a basket assembly that includes at least one spine 204. Like the previously described example, the spines 204 may have elliptical (e.g., circular) or rectangular (that may appear to be flat) cross-sections, and include a flexible, resilient material (e.g., a shape-memory alloy such as nickel-titanium, also known as Nitinol) forming a strut. Moreover, the spines 204 may be formed integrally with a crown 210, so as to form a unitary/monolithic spine framework. The crown 210 can share similar features as that of the previously described crown 110 to connect with an annulus 220. Further, proximal ends 230 of the spines 204 can include features similar to those of the previously described spines 104 to connect with a connecting hub 236.

Similarly, a connector tube 208 is connected to the crown 210. In some examples, the connector tube 208 is used to facilitate the expanding and collapsing of the spines 104. For example, the connector tube 208 can be translated along the longitudinal axis A1 to expand/collapse the spines 204. In the expanded configuration as well as the collapsed configuration (FIG. 10), one or more spines 204 can bow radially outwardly from the longitudinal axis A1. The spines 204, crown 210, and connector tube 208 are arranged generally along the longitudinal axis A1.

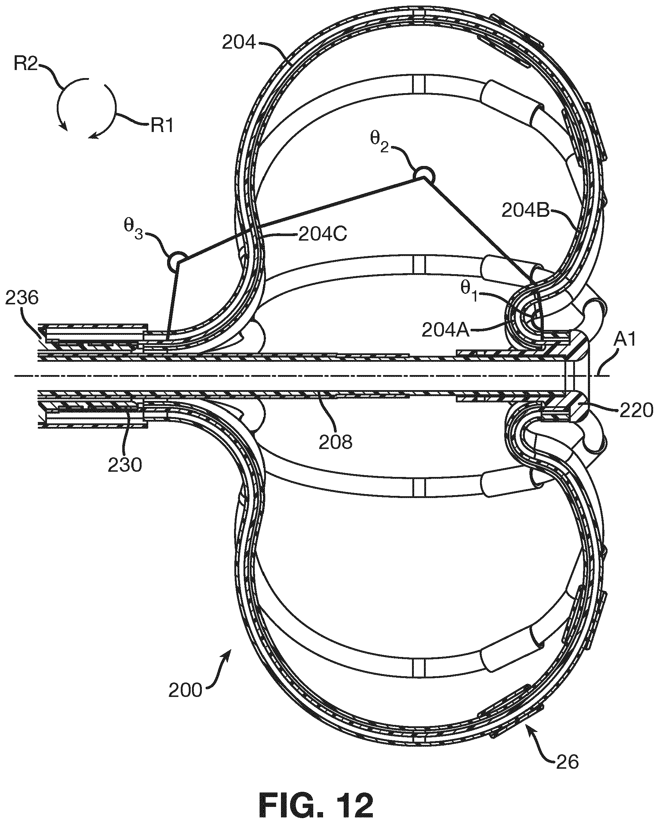

FIG. 12 is a schematic pictorial illustration showing a cross-sectional view of the end effector 200 cut relative to line 12-12 in FIG. 10. As shown, in a collapsed configuration (FIG. 12), the monolithic framework is generally spheroidal in shape. When the spines 204 are actuate to move to an expanded configuration via, for example, the connecting tube 208, the distal end of the framework is pulled towards the proximal end so as to form a petal-like structure. The petal structure allows for the electrodes 26 to ablate at the entry to the pulmonary vein, while the spheroidal structure allows for the electrodes 26 to ablate deep in the pulmonary vein.

To form the aforementioned spheroidal shape, each spine 204 has a series of bends. Specifically, each spine includes a first arcuate bend 204A, a second arcuate bend 204B, and a third arcuate bend 204C.

The first arcuate bend 204A extends from and is disposed proximal the crown 210 and is bent in a first radial direction R1 relative to the crown 210. The first radial direction R1 is about an axis that is perpendicular to the longitudinal axis A1. The first arcuate bend 204A is bent over a first angle θ₁. In some examples, the first angle θ₁ falls in the range of approximately one-hundred twenty degrees to approximately one-hundred eighty degrees.

The second arcuate bend 204B extends from the first arcuate bend 204A in a second radial direction R1. The second radial direction R2 is about the same axis as the first radial direction R1 that is perpendicular to the longitudinal axis A1, but in the opposite direction. The second arcuate bend 204B is bent over a second angle θ₂. In some examples, the second angle θ₂ falls in the range of approximately two-hundred forty degrees to approximately two-hundred seventy degrees.

The third arcuate bend 204C extends from the second arcuate bend 204B and is proximal the spine terminal ends 230. The third acuate bend 204C is bent in the first radial direction R1. The third arcuate bend 204C is bent over a third angle θ₃. In some examples, the third angle θ₃ falls in the range of approximately sixty degrees to approximately one-hundred fifty degrees.

The spines 204 further have a proximal half and a distal half, the dividing point being defined at approximately the center point (see where θ₂ is marked in FIG. 12) of the second arcuate bend 204B along the longitudinal axis A1. In this configuration, the electrodes 26 are disposed only on the distal half for the ablative purposes noted above.

Returning to the method 1000 discussed in FIG. 9, it will be appreciated that to form the end effector 200 depicted in FIGs. 10, the method 1000 may further include the steps of bending the plurality of spines 204 such that each spine 204 has a first arcuate bend 204A (Step 1006), bending the plurality of spines 204 such that each spine 204 has a second arcuate bend 204B (Step 1006), and bending the plurality of spines 204 such that each spine 204 has a third arcuate bend 204C (Step 1008). These steps can be performed prior to heat setting the spines 204, which biases the spines 204 to the spheroidal configuration.

The disclosed technology described herein can be further understood according to the following clauses:
Clause 1. A monolithic framework for an end effector of a medical device, the monolithic framework comprising cylindrical stock comprising: a crown extending along a longitudinal axis, defining a crown lumen, and defining a plurality of crown recesses configured to connect to an annulus; and a plurality of spines extending along the longitudinal axis and configured to bow outwardly relative to the longitudinal axis, each spine extending from the crown to a spine terminal end and being configured to connect to a connecting hub.
Clause 2. The monolithic framework of clause 1, consisting essentially of the cylindrical stock.
Clause 3. The monolithic framework of any one of clauses 1-2, each crown recess comprising: a first portion defined in a crown terminal end and extending parallel to the longitudinal axis; and a second portion extending from and generally perpendicular to the first portion.
Clause 4. The monolithic framework of clause 3, the crown further defining a plurality of crown holes extending generally perpendicular to the longitudinal axis, each crown hole being located between adjacent recesses of the plurality of crown recesses.
Clause 5. The monolithic framework of any one of clauses 1-4, each spine defining a plurality of spine recesses in at least one lateral side of the spine, the plurality of spine recesses being configured to interface with the connecting hub.
Clause 6. The monolithic framework of clause 5, each plurality of spine recesses comprising a first set of spine recesses defined in a first lateral side of the respective spine and a second set of spine recesses defined in a second lateral side of the respective spine.
Clause 7. The monolithic framework of clause 6, each spine defining at least one spine hole defined through the spine and extending perpendicular to the longitudinal axis, the at least one spine hole being positioned between the first set of spine recesses and the second set of spine recesses.
Clause 8. The monolithic framework of any one of clauses 1-7, each spine comprising: a first arcuate bend, proximal the crown, in a first radial direction perpendicular to the longitudinal axis; and a second arcuate bend, extending from the first arcuate bend, in a second radial direction perpendicular to the longitudinal axis.
Clause 9. The monolithic framework of clause 8, the first arcuate bend being bent at an angle that falls in the range of approximately one-hundred twenty degrees to approximately one-hundred eighty degrees.
Clause 10. The monolithic framework of any one of clauses 8-9, the second arcuate bend being bent at an angle that falls in the range of approximately two-hundred forty degrees to approximately two-hundred seventy degrees.
Clause 11. The monolithic framework of any one of clauses 8-9, each spine comprising: a third arcuate bend, proximal the spine terminal end and extending from the second arcuate bend, in the first radial direction.
Clause 12. The monolithic framework of clause 11, the third arcuate bend being bent at an angle that falls in the range of approximately sixty degrees to approximately one-hundred fifty degrees.
Clause 13. An end effector for a medical device, the end effector comprising: a monolithic framework comprising cylindrical stock that includes: a crown extending along a longitudinal axis and defining a plurality of crown recesses; and a plurality of spines extending along the longitudinal axis and configured to bow outwardly relative to the longitudinal axis, the plurality of spines being movable between a collapsed configuration and an expanded configuration; an annulus connected to the plurality of crown recesses; a connecting hub connected to the plurality of spines; and one or more electrodes connected to each spine of the plurality of spines.
Clause 14. The end effector of clause 13, the crown defining a crown lumen, and the annulus extending through the crown lumen.
Clause 15. The end effector of any one of clauses 13-14, the annulus comprising an atraumatic tip portion, a polygonal portion frictionally engaging an inner surface of the crown, and a tube portion.
Clause 16. The end effector of clause 15, the polygonal portion comprising a plurality of locking protrusions protruding radially outwards away from the longitudinal axis, each locking protrusion engaging in a respective crown recess of the plurality of crown recesses.
Clause 17. The end effector of clause 16, the polygonal portion defining a plurality of annulus holes, each annulus hole being positioned proximal a respective locking protrusion of the plurality of locking protrusions.
Clause 18. The end effector of clause 17, the crown defining a plurality of crown holes extending perpendicular to the longitudinal axis, each crown hole axially aligning with a respective annulus hole of the plurality of annulus holes.
Clause 19. The end effector of any one of clauses 13-18, further comprising a connector tube extending generally parallel to or coaxial with the longitudinal axis and connected to the annulus.
Clause 20. The end effector of clause 19, the annulus defining an annulus lumen, and the connector tube being connected to the annulus within the annulus lumen.
Clause 21. The end effector of any one of clauses 19-20, the connector tube being configured to move the plurality of spines between the collapsed configuration and the expanded configuration and/or route an irrigating fluid therethrough.
Clause 22. The end effector of any one of clauses 13-21, each spine of the plurality of spines having a shaped end defining at least one recess, the connecting hub defining a plurality of hub reliefs, and each hub relief being shaped to receive a respective shaped end of each spine.
Clause 23. The end effector of any one of clauses 13-22, each spine of the plurality of spines defining a spine hole, the connecting hub defining a plurality of connecting hub holes, and each spine hole axially aligning with a respective connecting hub hole of the plurality of connecting hub holes.
Clause 24. The end effector of any one of clauses 13-23, the plurality of spines comprising a generally spheroidal shape when in the collapsed configuration.
Clause 25. The end effector of clause 24, each spine of the plurality of spines having a proximal half and a distal half, the one or more electrodes being disposed on only the distal half.
Clause 26. A method of manufacturing a monolithic framework for a medical device, the method comprising: forming a generally cylindrical stock to include: a crown extending along a longitudinal axis, defining a crown lumen that is aligned with a stock lumen defined by the generally cylindrical stock, and defining a plurality of crown recesses configured to connect to an annulus; and a plurality of spines extending along the longitudinal axis, each spine extending from the crown to a spine terminal end and being configured to connect to a connecting hub; and heat setting the plurality of spines such that the plurality of spines bow outwardly relative to the longitudinal axis.
Clause 27. The method of clause 26, the forming step comprising at least one of: mechanical cutting, laser cutting, stamping, or combinations hereof.
Clause 28. The method of any one of clauses 26-27, further comprising: bending the plurality of spines such that each spine comprises: a first arcuate bend, proximal the crown, in a first radial direction perpendicular to the longitudinal axis; and a second arcuate bend, extending from the first arcuate bend, in a second radial direction perpendicular to the longitudinal axis.
Clause 29. The method of clause 28, further comprising: bending the plurality of spines such that each spine comprises: a third arcuate bend, proximal the spine terminal end and extending from the second arcuate bend, in the first radial direction.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A monolithic framework for an end effector of a medical device, the monolithic framework comprising cylindrical stock comprising:
a crown extending along a longitudinal axis, defining a crown lumen, and defining a plurality of crown recesses configured to connect to an annulus; and
a plurality of spines extending along the longitudinal axis and configured to bow outwardly relative to the longitudinal axis, each spine extending from the crown to a spine terminal end and being configured to connect to a connecting hub.

2. The monolithic framework of claim 1, consisting essentially of the cylindrical stock.

3. The monolithic framework of claim 1 or claim 2, each crown recess comprising:
a first portion defined in a crown terminal end and extending parallel to the longitudinal axis; and
a second portion extending from and generally perpendicular to the first portion.

4. The monolithic framework of claim 3, the crown further defining a plurality of crown holes extending generally perpendicular to the longitudinal axis, each crown hole being located between adjacent recesses of the plurality of crown recesses.

5. The monolithic framework of any preceding claim, each spine defining a plurality of spine recesses in at least one lateral side of the spine, the plurality of spine recesses being configured to interface with the connecting hub.

6. The monolithic framework of claim 5, each plurality of spine recesses comprising a first set of spine recesses defined in a first lateral side of the respective spine and a second set of spine recesses defined in a second lateral side of the respective spine, optionally each spine defining at least one spine hole defined through the spine and extending perpendicular to the longitudinal axis, the at least one spine hole being positioned between the first set of spine recesses and the second set of spine recesses.

7. The monolithic framework of any preceding claim, each spine comprising:
a first arcuate bend, proximal the crown, in a first radial direction perpendicular to the longitudinal axis; and
a second arcuate bend, extending from the first arcuate bend, in a second radial direction perpendicular to the longitudinal axis; and
optionally comprising:
a third arcuate bend, proximal the spine terminal end and extending from the second arcuate bend, in the first radial direction.

8. An end effector for a medical device, the end effector comprising:
a monolithic framework comprising cylindrical stock that includes:
a crown extending along a longitudinal axis and defining a plurality of crown recesses; and
a plurality of spines extending along the longitudinal axis and configured to bow outwardly relative to the longitudinal axis, the plurality of spines being movable between a collapsed configuration and an expanded configuration;
an annulus connected to the plurality of crown recesses;
a connecting hub connected to the plurality of spines; and
one or more electrodes connected to each spine of the plurality of spines.

9. The end effector of claim 8, the crown defining a crown lumen, and the annulus extending through the crown lumen.

10. The end effector of claim 8 or claim 9, the annulus comprising an atraumatic tip portion, a polygonal portion frictionally engaging an inner surface of the crown, and a tube portion.

11. The end effector of claim 10, the polygonal portion comprising a plurality of locking protrusions protruding radially outwards away from the longitudinal axis, each locking protrusion engaging in a respective crown recess of the plurality of crown recesses, optionally the polygonal portion defining a plurality of annulus holes, each annulus hole being positioned proximal a respective locking protrusion of the plurality of locking protrusions, further optionally the crown defining a plurality of crown holes extending perpendicular to the longitudinal axis, each crown hole axially aligning with a respective annulus hole of the plurality of annulus holes.

12. The end effector of any of claims 8 to 11, further comprising a connector tube extending generally parallel to or coaxial with the longitudinal axis and connected to the annulus.

13. The end effector of any of claims 8 to 12, each spine of the plurality of spines having a shaped end defining at least one recess, the connecting hub defining a plurality of hub reliefs, and each hub relief being shaped to receive a respective shaped end of each spine.

14. The end effector of any of claims 8 to 13, the plurality of spines comprising a generally spheroidal shape when in the collapsed configuration, optionally each spine of the plurality of spines having a proximal half and a distal half, the one or more electrodes being disposed on only the distal half.

15. A method of manufacturing a monolithic framework for a medical device, the method comprising:
forming a generally cylindrical stock to include:
a crown extending along a longitudinal axis, defining a crown lumen that is aligned with a stock lumen defined by the generally cylindrical stock, and defining a plurality of crown recesses configured to connect to an annulus; and
a plurality of spines extending along the longitudinal axis, each spine extending from the crown to a spine terminal end and being configured to connect to a connecting hub; and
heat setting the plurality of spines such that the plurality of spines bow outwardly relative to the longitudinal axis.
